# EUROPÄISCHE PATENTANMELDUNG

(11) **EP 1 438 988 A1**
(43) Veröffentlichungstag der Anmeldung: **21.07.2004**
(21) Anmeldenummer: 03000954.2
(22) Anmeldetag: 16.01.2003
(51) Int. Cl.: A61N 2/00, A61N 1/32, A61N 1/08

(54) **Therapiegerät und System zum Betreiben eines Therapiegeräts**

(71) Anmelder: ProCare Produktions- & Handels GmbH, 6911 Lochau b. Bregenz (AT)
(72) Erfinder: Schaknat, Carsten, 92358 Seubersdorf-Ittelhofen (DE); Imlauer, Georg, 6911 Lochau (AT)
(74) Vertreter: Schurack, Eduard F.

(57) **Zusammenfassung**

Die Erfindung betrifft ein Therapiegerät (1) zum Therapieren eines Patienten bezüglich verschiedener Indikationen, wobei das Therapiegerät (1) zumindest einen Speicher (13) , eine Schnittstelle zur Eingabe von Daten in den Speicher (13), eine Auswahleinheit (14), die mit dem Speicher (13)und einem Steuergerät (12) verbunden ist, aufweist, wobei das Steuergerät (12) zumindest eine Ausgabevorrichtung (15) ansteuern kann, über die Strom zur mittelbaren oder unmittelbaren Behandlung des Patienten ausgegeben werden kann, dadurch gekennzeichnet, dass in dem Speicher (13) jeder Indikation zumindest ein Satz an Anweisungen für das Steuergerät (12) zugeordnet ist, die Auswahleinheit (14) zumindest eine Indikation anzeigen kann und über die Auswahleinheit (14) zumindest eine Indikation ausgewählt werden kann. Weiterhin betrifft die Erfindung ein System für den Betrieb eines Therapiegeräts, das Strom zur mittelbaren oder unmittelbaren Behandlung des Patienten verwendet, dadurch gekennzeichnet, dass über eine Auswahleinheit (14) eine in einem Speicher abgelegte Liste von Indikationen angezeigt und eine Indikation ausgewählt werden kann, wobei durch die Auswahl der Indikation ein Satz an Anweisungen für ein Steuergerät (12), der der ausgewählten Indikation zugeordnet ist, aufgerufen wird und das Steuergerät (12) diese Anweisungen ausführt.

## Beschreibung

Die vorliegende Erfindung betrifft ein Therapiegerät, insbesondere ein Microstrom-Therapiegerät oder ein Magnetfeld-Therapiegerät, sowie ein System zum Betreiben eines Therapiegeräts.

Zur Behandlung verschiedener Erkrankungen und/oder Beschwerden von Patienten hat sich herausgestellt, dass durch Nachahmung des bioelektrischen Systems der natürliche Ausgleich gestörter Zellsysteme erreicht werden kann und damit Heilungsprozesse eingeleitet und unterstützt werden können. Weiterhin ist erkannt worden, dass durch Erzeugung eines elektromagnetischen Biofelds bioenergetisch lebenswichtige Signale für den Menschen nachgebildet werden können, die einer Behandlung und Heilung verschiedener Indikationen dienen können.

Bei Therapiegeräten, bei denen Strom unmittelbar, beispielsweise in Form von Microstrom, oder mittelbar, beispielsweise durch Erzeugung eines Magnetfelds, zur Behandlung des Patienten verwendet wird, ist es notwendig, verschiedene Variablen des Stroms gezielt einzustellen, um die gewünschten Ergebnisse zu erhalten und eine Verletzung des Patienten zu verhindern. Bei der Behandlung durch Microstrom sind beispielsweise bestimmte Frequenzen einzustellen, um Nervenfasern mit bestimmten Durchmessern und dementsprechend bestimmten Funktionen ansprechen zu können. Weiterhin ist die Intensität des Stroms insofern für eine erfolgreiche Behandlung durch Microstrom von Bedeutung als dadurch der axoplasmatische Transport innerhalb von Nervenzellen beeinflußt wird. Ein erfahrener Therapeut kann diese Variablen, sowie weitere Parameter gezielt an Therapiegeräten einstellen und eine erfolgreiche Behandlung ohne die Gefahr der Verletzung des Patienten sicherstellen. Zur Zeit sind außerdem Therapiegeräte bekannt, bei denen unterschiedliche voreingestellte Programme von dem Patienten aufgerufen werden können. Der Nachteil dieser bekannten Therapiegeräte, bei denen die Variablen einzeln einzustellen sind, liegt darin, dass eine gezielte Behandlung einzelner Indikationen für den Patienten mangels Fachkenntnis selber nicht möglich ist. Bei Programmen, die in den Geräten vorprogrammiert sind, muss der Patient das Programm wählen, dass nach seiner Auffassung eine Hilfestellung für seinen Indikationsbereich bieten könnte. Dies ist in der Regel aufgrund mangelnde Therapie-Kenntnis des Patienten schwierig und kann zu falschen Behandlungen führen.

Die Aufgabe der vorliegenden Erfindung ist es daher, ein sicheres und bedienerfreundliches Therapiegerät und System zum Betreiben eines Therapiegeräts zu schaffen.

Diese Aufgabe wird erfindungsgemäß durch ein Therapiegerät zum Therapieren eines Patienten bezüglich verschiedener Indikationen gelöst, wobei das Therapiegerät zumindest eine Speichereinheit, eine Schnittstelle zur Eingabe von Daten in den Speicher, eine Auswahleinheit, die mit dem Speicher und einem Steuergerät verbunden ist, aufweist, wobei das Steuergerät zumindest eine Ausgabevorrichtung ansteuern kann, über die Strom zur mittelbaren oder unmittelbaren Behandlung des Patienten ausgegeben werden kann. Das erfindungsgemäße Therapiegerät zeichnet sich dadurch aus, dass in der Speichereinheit jeder Indikation zumindest ein Satz an Anweisungen für das Steuergerät zugeordnet ist, die Auswahlvorrichtung zumindest eine Indikation anzeigen kann und über die Auswahleinheit zumindest eine Indikation ausgewählt werden kann.

Durch die Zuordnung zumindest eines Satzes an Anweisungen für das Steuergerät zu einer Indikation können für die Behandlung dieser spezifischen Indikation notwendige Variablen präzise voreingestellt werden. Da die Auswahlvorrichtung die Indikation anzeigen kann und der Benutzer, insbesondere der Patient, eine Indikation auswählen kann, wird so die für die entsprechende Indikation geeignete Behandlung sichergestellt. Das erfindungsgemäße Therapiegerät erleichtert somit die Bedienung für den Patienten, und die Gefahr einer falschen Einstellung des Geräts bzw. einer falschen Behandlung wird vermieden.

Gemäß einer Ausführungsform kann die Zuordnung von Anweisungen für das Steuergerät zu einer Indikation durch eine Eingabevorrichtung erfolgen. Diese Eingabevorrichtung kann Teil des Therapiegeräts sein, oder lösbar mit dieser verbunden werden. Bevorzugt wird die Eingabevorrichtung ein separates Gerät darstellen, das mit der Schnittstelle zur Eingabe von Daten in den Speicher des Therapiegeräts verbunden werden kann. Ein solches Eingabegerät kann beispielsweise von einem Therapeuten bedient werden, der die einzustellenden Anweisungen an das Steuergerät den Indikationen zuweisen kann. Alternativ kann eine Liste von Indikationen mit diesen zugewiesenen Anweisungssätzen bei der Herstellung des Therapiegeräts in diesem vorprogrammiert werden. Der Vorteil einer Eingabevorrichtung, über die die Zuordnung der Anweisungssätze zu den Indikationen erfolgen kann, liegt darin, dass ein Therapeut diese Zuweisungen personenspezifisch durchführen kann. Je nach Krankheitsbild, Alter und dergleichen können zur Behandlung einer gewissen Indikation unterschiedliche Variablen notwendig sein. Über die Eingabevorrichtung kann zusätzlich ein Katalog von Indikationen in den Speicher des Therapiegeräts eingespeist werden. Alternativ kann dieser Katalog an Indikationen bereits bei der Herstellung des Therapiegeräts vorprogrammiert werden.

Vorzugsweise stellt das Therapiegerät ein Microstromtherapiegerät dar, das zumindest einen Generator zur Erzeugung eines Microstroms umfasst und bei dem die Ausgabevorrichtung zur Ausgabe von Microstrom ausgelegt ist. Vorzugsweise wird durch das Therapiegerät ein Microstrom mit einer Intensität von 5 bis 800µA, vorzugsweise 10 bis 500µA erzeugt. Insbesondere bei Microstromtherapiegeräten ist die Zuordnung von Anweisungen für das Steuergerät zu spezifischen Indikationen von besonderer Bedeutung, da bei der Behandlung durch Microstrom eine sehr genaue Einstellung von Variablen von besonderer Bedeutung ist, um die gewünschten Behandlungseffekte zu erzielen. Die Ausgabevorrichtung können beispielsweise Pads darstellen, die lösbar mit dem Therapiegerät verbunden werden können, und auf die Haut aufgebracht werden können.

Alternativ oder zusätzlich kann zumindest ein Teil des Therapiegeräts zur Erzeugung eines Magnetfelds ausgelegt sein, das über die Ausgabevorrichtung ausgegeben werden kann. Bei einem solchen Magnetfeldtherapiegerät ist ebenfalls die präzise Einstellung von Variablen von besonderer Bedeutung.

In einer bevorzugten Ausführungsform umfasst der Satz an Anweisungen an das Steuergerät Informationen über die Frequenz, die Intensität, die Polarität und die Übertragungswelle des Stroms, der zur Behandlung des Patienten dienen soll. Durch die Zuordnung von Anweisungen bezüglich dieser Variablen kann eine falsche Behandlung mittels des Therapiegeräts vermieden werden.

Zusätzlich oder alternativ kann der Satz an Anweisungen an das Steuergerät Informationen über die Dauer der Ausgabe des Stroms umfassen. Auch diese Variable ist für eine erfolgreiche Behandlung und die Vermeidung der Verletzung des Patienten von großer Bedeutung. Bei zu langer Anwendung von Therapiegeräten, die mittelbar oder unmittelbar Strom zur Behandlung des Patienten verwenden, kann es zu Verletzungen kommen und bei zu kurzer Behandlung kann kein Erfolg erzielt werden.

Gemäß einem weiteren Aspekt wird die Aufgabe, die der vorliegenden Erfindung zugrunde liegt, durch ein System für den Betrieb eines Therapiegeräts, das Strom zur mittelbaren oder unmittelbaren Behandlung des Patienten verwendet, gelöst. Das System zeichnet sich dadurch aus, dass über eine Auswahleinheit eine in einem Speicher abgelegte Liste von Indikationen angezeigt und eine Indikation ausgewählt werden kann, wobei durch die Auswahl der Indikation ein Satz an Anweisungen für ein Steuergerät, der der ausgewählten Indikation zugeordnet ist, aufgerufen wird und das Steuergerät diese Anweisungen ausführt. Durch dieses System für den Betrieb eines Therapiegeräts wird die Interaktion mit dem Patienten auf ein Minimum reduziert. Das Einstellen von kritischen Variablen, wie beispielsweise der Frequenz und/oder der Intensität des Stroms durch den Patienten kann entfallen. Somit wird durch dieses System die Heimanwendung eines Therapiegeräts, das Strom zur mittelbaren oder unmittelbaren Behandlung des Patienten verwendet, ohne Gefahr und effektiv möglich.

In einer Ausführungsform kann die in dem Speicher abgelegte Liste von Indikationen eine hierarchische Struktur aufweisen, die Indikationen in Indikationsbereiche zusammenfasst. Durch diese Art der Katalogisierung von Indikationen kann es für den Patienten erleichtert werden, die seinem Krankheitsbild entsprechende Indikation aufzufinden. Hierbei kann zunächst ein Indikationsbereich gewählt werden, der dann eine Liste von Indikationen in diesem Bereich anzeigt.

Vorzugsweise ist der Zugriff auf den Speicher zur Eingabe und/oder Änderung der gespeicherten Daten über eine Zugriffssicherung gesichert. Diese Zugriffssicherung kann in Form einer Verschlüsselung der gespeicherten Daten ausgeführt sein. Für den Zugriff dieser verschlüsselten Daten kann ein Code benutzt werden, wodurch ein Zugriff durch Unberechtigte vermieden werden kann. Insbesondere kann durch diese Zugriffssicherung verhindert werden, dass ein Patient die gespeicherten Daten ändert und dadurch die Gefahr der Verletzung bzw. das Ausbleiben des Therapieerfolges hervorruft. Ein Code, der die Zugriffssicherung darstellen kann, sollte vorzugsweise ausschließlich durch einen geschulten Therapeuten eingegeben werden können.

Das erfindungsgemäße System ist vorzugsweise für den Betrieb eines Therapiegeräts gemäß der vorliegenden Erfindung ausgelegt.

Die Merkmale und Vorteile, die in Bezug auf das Therapiegerät erläutert werden, gelten entsprechend und soweit anwendbar für das erfindungsgemäße System und umgekehrt.

Im Folgenden wird die Erfindung anhand der beiliegenden Figuren detaillierter erläutert. Es zeigen:
- Figur 1:: eine schematische Blockansicht eines erfindungsgemäßen Therapiegeräts;
- Figur 2:: eine schematische Blockansicht der Speicheraufteilung in einem erfindungsgemäßen Therapiegerät und
- Figur 3:: eine schematische Darstellung einer Auswahleinheit.

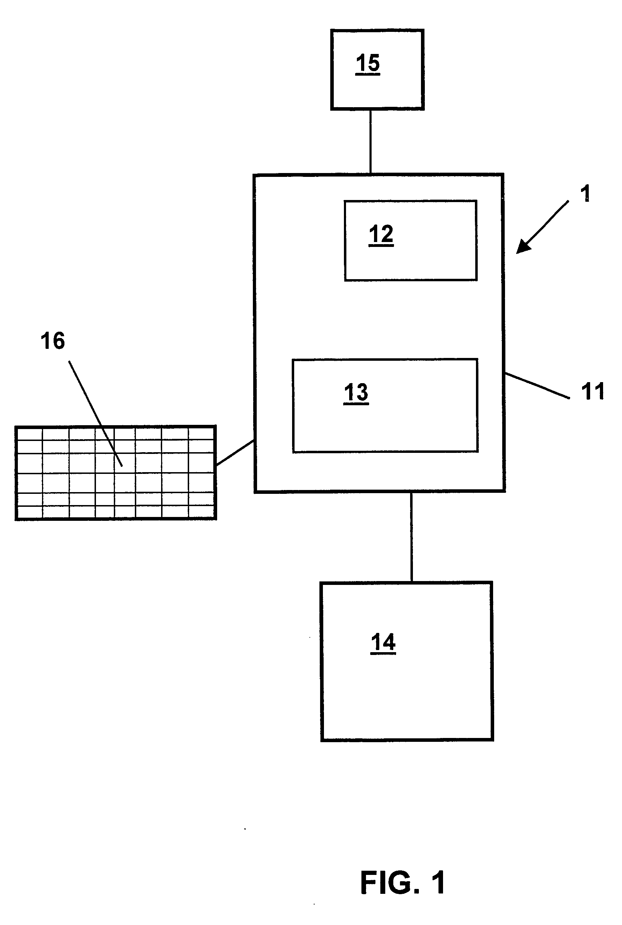

In Figur 1 ist eine Ausführungsform eines erfindungsgemäßen Therapiegeräts 1 schematisch in Blockdarstellung gezeigt. In einem Gehäuse 11 ist ein Steuergerät 12 sowie ein Speicher 13 vorgesehen. In der dargestellten Ausführungsform ist mit dem Gehäuse 11 verbunden eine Ausgabevorrichtung 15 dargestellt. Weiterhin ist eine Auswahleinheit 14 ebenfalls außerhalb des Gehäuses 11, aber mit diesem verbunden, angedeutet. Schließlich ist in Figur 1 eine Eingabevorrichtung 16 gezeigt, die mit dem Gehäuse 11 und insbesondere mit dem in dem Gehäuse 11 befindlichen Speicher 13 verbunden werden kann.

Die Funktionsweise eines erfindungsgemäßen Therapiegeräts sowie eines erfindungsgemäßen Systems zum Betreiben des Therapiegeräts wird nunmehr unter Bezugnahme auf Figur 1 beschrieben.

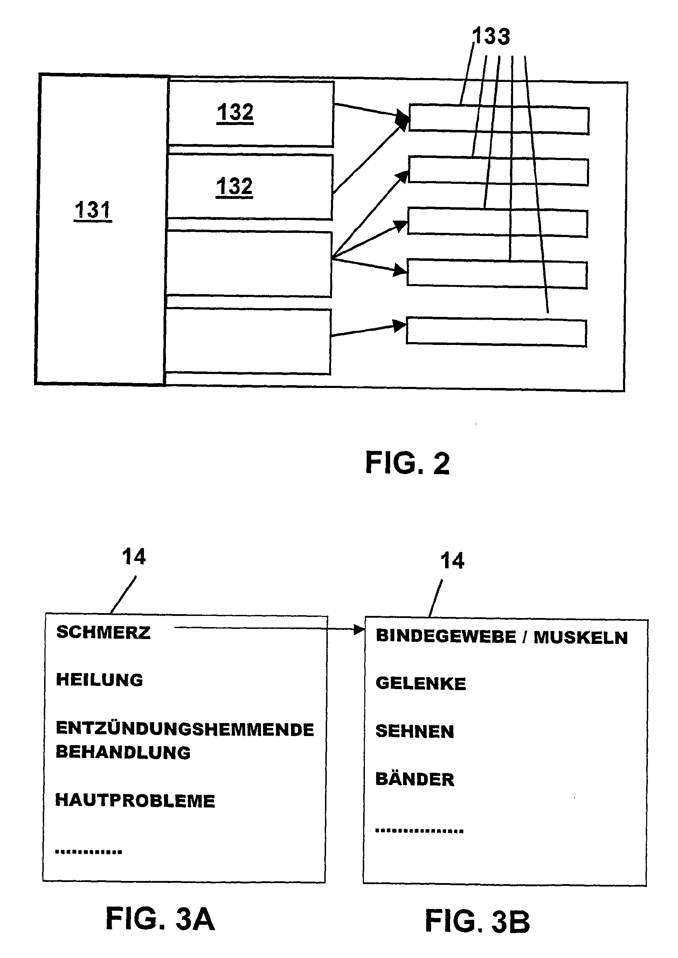

Über die Eingabevorrichtung 16 können in den Speicher 13 Daten und Informationen eingegeben bzw. darin abgespeicherte Daten und Informationen geändert werden. Vorzugsweise wird von einem Arzt oder Therapeuten über die Eingabevorrichtung 16 ein Katalog von Indikationen in den Speicher 13 abgelegt. Die Indikationen, die in dem Katalog enthalten sind, werden weiterhin, vorzugsweise ebenfalls über die Eingabevorrichtung 16, mit weiteren Daten, die Anweisungen an das Steuergerät darstellen, in dem Speicher 13 in Bezug gebracht. Die Speicherung der Daten wird später unter Bezug auf Figur 2 genauer beschrieben. Wird ein so vorprogrammiertes Therapiegerät 1 von einem Patienten bedient, so kann sich dieser mittels der Auswahleinheit 14 zunächst die in dem Speicher 13 abgelegten Indikationen anzeigen lassen. Aus diesen kann der Patient die für seine Beschwerden zutreffende Indikation auswählen. Von der Auswahleinheit wird diese gewählte Indikation an das Steuergerät 12 geleitet, das daraufhin aus dem Speicher 13 die Anweisungen, die dieser Indikation zugeordnet sind, ausliest. Entsprechend der ausgelesenen Anweisungen stellt das Steuergerät 12 Variablen ein, die insbesondere Steueranweisungen an die Ausgabevorrichtung 15 sein können. Zusätzlich oder alternativ kann das Steuergerät beispielsweise einen Generator (nicht dargestellt) ansteuern, über den ein Strom bzw. ein Magnetfeld erzeugt wird. Das Steuergerät 1 kann als Stromquelle eine Batterie, insbesondere eine wiederaufladbare Batterie verwenden, oder an ein Stromnetz angeschlossen werden.

In Figur 2 ist schematisch die Datenstruktur, der in dem Speicher 13 abgelegten Daten angedeutet. In einem ersten Bereich 131 sind Indikationsbereiche abgelegt. Jedem dieser Indikationsbereiche sind mehrere Einzelindikationen 132 untergeordnet. Zu diesen Einzelindikationen sind Sätze von Anweisungen 133 zugeordnet.

In Figur 3 ist eine mögliche Anzeige an der Auswahleinheit 14 dargestellt. Schaltet der Benutzer das Therapiegerät 1 an, so kann beispielsweise eine Auflistung von Indikationsbereichen, wie in Figur 3A angedeutet angezeigt werden. Indikationsbereiche können beispielsweise Schmerz, Heilung, entzündungshemmende Behandlung, Hauptprobleme, Relaxation, craniale Behandlung, Immunstimulation, antibakterielle Behandlung, Magen-Darm-Probleme, Nervenregeneration, Lymphdrainage, Ästhetikprogramm sein. Wählt der Patient beispielsweise den Indikationsbereich Schmerz aus, so wird an der Auswahleinheit 13 die Liste der Indikationen, die diesem Indikationsbereich zugehören, angezeigt. Eine solche Anzeige ist in Figur 3B beispielhaft dargestellt. Einzelindikationen können beispielsweise Bindegewebe/Muskeln, Gelenke, Sehnen oder Bänder sein. Wählt der Benutzer eine dieser Indikationen beispielsweise Sehnenschmerz aus, so wird über das Steuergerät 12 der dieser Indikation zugeordnete Satz an Anweisungen aus dem Speicher 13 ausgelesen. Der Satz der Anweisungen, der für die entsprechende Indikation in dem Speicher 13 vorprogrammiert ist, umfasst insbesondere die Frequenz, die Intensität, die Polarität, sowie die Übertragungswelle des zu verwendenden Stromes und bestimmt die Behandlungszeit. Die Frequenz kann beispielsweise im Bereich von 0,01 bis 1000,00 Hz auf 1/100 genau vorprogrammiert werden. Die Intensität wird vorzugsweise im Bereich von 10 bis 500µA festgelegt. Bei der Polarität des Stromes kann monopolar positiv, monopolar negativ oder alternierend vorprogrammiert sein, wobei die Polarität bei der alternierenden Einstellung frequenzabhängig, beispielsweise ca. alle 3 Sekunden, wechselt. Die Übertragungswelle stellt vorzugsweise eine modulierbare Rechteckwelle dar. Die Behandlungszeit, die für die einzelnen Indikationen vorprogrammiert sein kann, kann im Bereich von 30 Minuten bis zu 8 Stunden liegen.

Die vorliegende Erfindung ist nicht auf die dargestellten Ausführungsformen beschränkt. Die Ausgabevorrichtung des Therapiegeräts kann beispielsweise Pads darstellen, die an der Haut des Patienten befestigt werden können. Diese Art der Ausgabevorrichtung bietet sich bei Microstromtherapiegeräten an. Alternativ oder zusätzlich kann die Ausgabevorrichtung in dem Gehäuse des Therapiegeräts aufgenommen sein und beispielsweise für die Erzeugung und Ausgabe eines Magnetfelds dienen.

Auch die Auswahleinheit kann, anders als in den Figuren dargestellt, in dem Gehäuse aufgenommen sein und beispielsweise lediglich einen Bildschirm an diesem Gehäuse darstellen, über den eine Auswahl erfolgen kann. Die Eingabevorrichtung, über die Daten in das Therapiegerät, insbesondere in den Speicher des Therapiegeräts eingelesen bzw. geändert werden können, kann ein separates Gerät darstellen, das lediglich in das Therapiegerät eingesteckt wird, während die notwendigen Daten übertragen werden. Weiterhin ist es möglich, dass die Auswahleinheit unmittelbar mit der Speichereinheit zusammenwirkt, so dass vom Speicher die Sätze an Anweisungen ausgelesen werden, die der ausgewählten Indikation zugeordnet sind, ohne dass das Steuergerät zwischengeschaltet ist.

Als Sätze von Anweisungen können neben einzelnen Werten für beispielsweise die Intensität oder die Frequenz auch Bereiche vorprogrammiert sein. So können beispielsweise nicht nur jede einzelne Frequenz im Bereich von 0,01 bis 1000 Hz individuell angesteuert werden, sondern es sind auch frequenzüberlagernde Therapien und durch galvanische Trennung der Ausgangskanäle Therapien mit Vektoreffekt möglich. Weiterhin ist es möglich, dass der Satz von Anweisungen bezüglich der Übertragungswelle Informationen enthält, so dass diese insbesondere im Bereich der ersten Sekunde der Therapie, insbesondere der Stimulation, individuell modulierbar ist. Diese individuelle Modulation kann beispielsweise durch den Therapeuten ebenfalls in dem Speicher personenspezifisch einprogrammiert sein. Eine individuelle Programmierung des Magnetfeld-Therapiegeräts, beispielsweise mit homöopathischen Informationen ist ebenfalls möglich.

Die Indikationsbereiche, die bei einer Ausführungsform der Erfindung zunächst über die Auswahleinheit ausgewählt werden können, können entweder einzelne Indikationen oder Indikationsbereiche zusammenfassen. So ist es beispielsweise möglich, als Indikationsbereiche medizinische Anwendungen, medikamentöse Anwendungen, Massage und dergleichen vorzuprogrammieren. Dem Indikationsbereich "medizinische Anwendung" kann dann beispielsweise der weitere Indikationsbereich "Schmerztherapie" zugeordnet sein, der sich in die einzelnen Indikationen Muskelschmerzen, Sehnenschmerzen etc. unterteilt. Erfindungsgemäß sind vorzugsweise den einzelnen Indikationen Sätze von Anweisungen für das Steuergerät zugeordnet. Es liegt aber auch im Rahmen der Erfindung einen Indikationsbereich einem Satz von Anweisungen zuzuordnen.

Mit dem erfindungsgemäßen Therapiegerät und dem erfindungsgemäßen System wird daher die Möglichkeit für einen Patienten geschaffen, eine Therapie, wie beispielsweise eine Microstromtherapie, oder Magnetfeldtherapie ohne permanente ärztliche bzw. therapeutische Beobachtung durchführen zu können. Weiterhin bietet das erfindungsgemäße Therapiegerät und das erfindungsgemäße System einen hohen Grad an Sicherheit gegen falsche Behandlung, die gegebenenfalls zu Verletzungen, oder zumindest zu verminderten Behandlungsergebnissen führen kann. Besonders bevorzugt werden die in dem Speicher des Therapiegerät abgelegten Daten personenspezifisch definiert. Dies kann insbesondere durch einen Therapeuten bzw. einen Arzt erfolgen. Gemäß der Erfindung kann ein Zugriff für andere Personen, beispielsweise mittels eines Codes, vermieden werden.

## Patentansprüche

1. Therapiegerät (1) zum Therapieren eines Patienten bezüglich verschiedener Indikationen, wobei das Therapiegerät (1) zumindest einen Speicher (13) , eine Schnittstelle zur Eingabe von Daten in den Speicher (13), eine Auswahleinheit (14), die mit dem Speicher (13)und einem Steuergerät (12) verbunden ist, aufweist, wobei das Steuergerät (12) zumindest eine Ausgabevorrichtung (15) ansteuern kann, über die Strom zur mittelbaren oder unmittelbaren Behandlung des Patienten ausgegeben werden kann, **dadurch gekennzeichnet, dass** in dem Speicher (13) jeder Indikation zumindest ein Satz an Anweisungen für das Steuergerät (12) zugeordnet ist, die Auswahleinheit (14) zumindest eine Indikation anzeigen kann und über die Auswahleinheit (14) zumindest eine Indikation ausgewählt werden kann.

2. Therapiegerät nach Anspruch 1, **dadurch gekennzeichnet, dass** die Zuordnung des mindestens eines Satzes an Anweisungen für das Steuergerät (12), zu der jeweiligen Indikation durch eine Eingabevorrichtung (16) erfolgt.

3. Therapiegerät nach einem der Ansprüche 1 oder 2, **dadurch gekennzeichnet, dass** das Therapiegerät (1) zumindest einen Generator zur Erzeugung eines Microstroms umfasst und die Ausgabevorrichtung (15) zur Ausgabe von Mirkostrom ausgelegt ist.

4. Therapiegerät nach Anspruch 3, **dadurch gekennzeichnet, dass** in dem Therapiegerät ein Mircostrom mit einer Intensität von 5 bis 800µA, vorzugsweise 10-500µA erzeugt werden kann.

5. Therapiegerät nach einem der Ansprüche 1 bis 4, **dadurch gekennzeichnet, dass** zumindest ein Teil des Therapiegeräts (1) zur Erzeugung eines Magnetfelds ausgelegt ist, das über die Ausgabevorrichtung (15) ausgegeben werden kann.

6. Therapiegerät nach einem der Ansprüche 1 bis 5, **dadurch gekennzeichnet, dass** der Satz an Anweisungen an das Steuergerät (12) Informationen über die Freuquenz, die Intensität, die Polarität und die Übertragungswelle des Stroms, der zur Behandlung des Patienten dienen soll, umfasst.

7. Therapiegerät nach einem der Ansprüche 1 bis 6, **dadurch gekennzeichnet, dass** der Satz an Anweisungen an das Steuergerät (12) Informationen über die Dauer der Ausgabe des Stroms umfasst.

8. System für den Betrieb eines Therapiegeräts, das Strom zur mittelbaren oder unmittelbaren Behandlung des Patienten verwendet, **dadurch gekennzeichnet, dass** über eine Auswahleinheit (14) eine in einem Speicher abgelegte Liste von Indikationen angezeigt und eine Indikation ausgewählt werden kann, wobei durch die Auswahl der Indikation ein Satz an Anweisungen für ein Steuergerät (12), der der ausgewählten Indikation zugeordnet ist, aufgerufen wird und das Steuergerät (12) diese Anweisungen ausführt.

9. System nach Anspruch 8, **dadurch gekennzeichnet, dass** die in dem Speicher abgelegte Liste von Indikationen eine hirarchische Struktur aufweist, die Indikationen in Indikationsbereiche zusammenfasst.

10. System nach einem der Ansprüche 8 oder 9, **dadurch gekennzeichnet, dass** der Zugriff auf den Speicher (13) zur Eingabe und / oder Änderung der gespeicherten Daten über eine Zugriffssicherung gesichert ist.

11. System nach Anspruch 8 bis 10, **dadurch gekennzeichnet, dass** dieses für den Betrieb eines Therapiegeräts (1) nach einem der Ansprüche 1 bis 7 ausgelegt ist.
